# EUROPÄISCHE PATENTSCHRIFT

(11) **EP 0 334 243 B1**
(45) Veröffentlichungstag und Bekanntmachung des Hinweises auf die Patenterteilung: **20.01.1993**
(21) Anmeldenummer: 89104866.2
(22) Anmeldetag: 18.03.1989
(51) Int. Cl.: A61K 31/44, A61K 31/47

(54) **Arzneimittel enthaltend Aminoacridine zur iontophoretischen Behandlung von Carcinomen**
Medicament comprising amino acridines in the iontophoretic treatment of cancer
Médicament contenant des amines d'acridine pour le traitement iontophorétiques des carcinomes

(30) Priorität: 23.03.1988 DE 3809814; 10.06.1988 DE 3819842
(43) Veröffentlichungstag der Anmeldung: 27.09.1989
(73) Patentinhaber: Thiel, K.H., Dr., D-74074 Heilbronn (DE)
(72) Erfinder: Thiel, K.H., Dr., D-74074 Heilbronn (DE)
(74) Vertreter: Reitzner, Bruno, Dr.

(56) Entgegenhaltungen:
- FR-A- 2 509 182
- GB-A- 2 076 288
- Arch. Dermatol. Forsch., Vol. 243, Nr. 2, 1972, S. 107 - 113
- Chem.-Biol. Interact., Vol. 42, Nr. 1, Oktober 1982, S. 97 - 105
- Yale J. Biol. Med., Vol. 46, Nr. 5, Dezember 1973, S. 464 - 470
- Mol. Pharmacol., Vol. 20, Nr. 2, September 1981, S. 404 - 414
- Anti-Cancer Drug Des., Vol. 1, Nr. 4, April 1987, S. 281 - 289
- Biopolymers, Vol. 11, Nr. 12, 1972, S. 2439 - 2459
- Biochem. Biophys. Res. Commun., Vol. 70, Nr. 3, Juni 1976, S. 824 - 831
- Stud. Biophys., Vol. 35, Nr. 1, 1973, S. 57 - 58
- Pharm. Acta Helv., Vol. 51, Nr. 6, 1976, S. 160 - 163
- J. Clin. Hosp. Pharm., Vol. 8, Nr. 4, 1983, S. 345 - 348
- Stud. Biophys., Vol. 24 - 25, 1970, S. 233 - 240
- Cancer Treatmendt reports, vol 69, no. 2, February 1985, Seiten 189-94. M. Al-Sarrraf et al.: "Phase II trial of cyclophosphamide, doxorubicin, and cisplatin (CAP) versus amsacrine in patents with transitional cell carcinoma of the urinary bladder: a southwest oncology group study".
- Cancer treatment reports, vol 67, no 4, April 1983, Seiten 391-2, R.B. Natale et al.: "Phase II trial of amsacrine (AMSA) in urinary bladder cancer".
- The Merck Index 10 Ed. 1983, Encyclopedia of Chemicals, Durgs and Biologicals, Seiten 86,87; Merck & Co, Rahway US
- Pharmazeutiche Wirkstoffe, Band 5, Seite 1247, 1987 Thieme Verlag Stuttgart, DE, "Lokalanaestheticum zur Varizenverödung"
- Angew. Chem. Band 62, Seiten 7-9, 1950. C. Schöller:"Uber die Regelung der Wasser- und Lipoidlöslichkeit pharmazeutisch werksamer Verbindungen"

## Beschreibung

Die Erfindung betrifft die Verwendung gewisser Heterocyclen zur Herstellung von Arzneimitteln für die iontophoretische Behandlung maligner Tumoren.

Es ist bekannt, daß bestimmte Heterocyclen, d.h. Acridin-, Chinolin-und Pyridinderivate mit einem Ringstickstoffatom und mindestens einer außerhalb der Ringstruktur stehenden Aminogruppe, die mit dem Ringstickstoffatom eine tautomere Diiminstruktur ausbilden kann, Mitosegifte darstellen (vgl. Lettré H.: Über Mitosegifte. Ergebnisse der Physiologie, 46, 379-452 (1950)).

Einige dieser Heterocyclen mit zwei oder mehreren kondensierten aromatischen Ringsystemen haben die Fähigkeit, sich in die Doppelhelix der Desoxyribonucleinsäure (DNA) von Bakterien oder Gewebezellen derart einzuschieben, daß sowohl die Replikation als auch die Transkription nicht mehr ordnungsgemäß ablaufen können. Diese Moleküle bezeichnet man als Interkalantien (vgl. D. Schmäl, Arzneimittelforschung, 21. Beiheft, 3. Auflage (1981), s. 424). Die biologische Interaktion dieser Heterocyclen, insbesondere der Acridinverbindungen, beruht wahrscheinlich auf einer Interkalation mit der DNA durch Anlage bzw. Einlage zwischen die Basenpaarschichten, was durch die flache Struktur dieser Heterocyclen bedingt ist. Hierbei werden die Guanin-Cytosin-Basenpaare bevorzugt, wobei wahrscheinlich die Bildung eines Komplexes mit DNA und einem Reparatur-Enzym erfolgt. Die Aktivität der Polymerase wird reduziert, und die DNA kann nicht entrollt werden. Ferner zerbricht die DNA-Helix in Einzel- und Doppelstränge mit konsekutiven DNA-Protein-Bindungen.

Aus der EP-00 35 862-A2 ist die Verwendung von 4'-(9-Acridinylamino)-Methansulfon-m-Anisidid als antineoplastisches Mittel bekannt. Es handelt sich hierbei jedoch nicht um ein Aminoacridin, das in einer tautomeren Diiminform vorliegt. Die Verbindung ist extrem toxisch, deshalb kaum anwendbar. Sie war bei gewissen soliden Tumoren so geringfügig wirksam, daß sie nicht in ein klinisch-therapeutisches Programm aufgenommen wurde. Sie wurde insbesondere nicht zur Behandlung des Blasen-Carcinom angewendet und hat ihre bevorzugte Indikation bei der Leukämie. Eine lokale Anwendung ist durch ihre gewebsschädigende Wirkung ausgeschlossen.

Die CH-647 677 betrifft ein pharmazeutisches Gemisch, enthaltend 4'-(9-Acridinylamin)-Methansulfon-m-Anisidid in Mischung mit Milchsäure im molaren Verhältnis von 1,5:1 bis 4:1. Es handelt sich also um dasselbe Acridinderivat wie es in der EP-00 35 862-A2 beschrieben ist, wobei durch die Salzbildung mit Milchsäure die Wasserlöslichkeit der Wirksubstanz erhöht werden soll, so daß sie zur intravenösen Verabreichung geeignet ist. Möglich ist auch eine orale Verabreichung. Es handelt sich also um ein systemisches Arzneimittel.

In der Literaturstelle "THE MERCK INDEX", 1983 sind Acriflavine, Proflavine und Streptonigrin genannt, die als anti-infektiv bzw. antiseptisch wirksame Arzneimittel bezeichnet sind. Das Streptonigrin wurde auch als Antineoplastikum bezeichnet. Es finden sich jedoch keine Hinweise auf die Anwendung dieser Verbindungen bei der Behandlung von soliden Tumoren, insbesondere beim Blasencarcinom.

Aus der Literaturstelle CA 84: 117676a ist bekannt, daß durch Proflavinsulfat und Ethidiumbromid die t-RNA-Nucleotidyltransferase von Ehrlich'schen Tumorzellen gehemmt wird. Bei diesen Zellen handelt es sich jedoch nur um Testzellen für Mitosehemmer, so daß nicht vorhersehbar war, wie sich das Proflavin bei soliden Tumoren, insbesondere bei Blasencarcinomen, verhalten würde.

Aus der Literaturstelle "Folia Vetennavia", 31,1 (1987), Seiten 135 bis 140 (CA 108: 87713e) ist bekannt, daß Colchicin zur iontophoretischen Behandlung von Tumoren verwendet werden kann, die durch ASV (Avian Sarcoma Virus) induziert wurden. Es wurden Hühner behandelt. Bei Colchicin handelt es sich jedoch nicht um einen Heterocyclus mit Aminfunktionen, die in einer tautomeren Diiminform vorliegen können. Das Colchicin ist ein starkes Mitosegift, weshalb es in der humanen Krebstherapie bisher noch keinen Eingang gefunden hat.

Aus der Literaturstelle Arch.Dermatol.Forsch., Vol. 243, Nr. 2, 1972, S. 107-113 ist die Hemmwirkung von Proflavin auf den Einbau von DNS-, RNS- und Proteinvorstufen in Hamstermelanom bekannt. Bei der Injektion von Proflavin, (10mg/kg Körpergewicht) und Wiederholung dieser Dosis nach 24 h ist eine Abnahme der HUMP-Inkorporation um 37% in den Zellkernen und um 17% in den Mitochondrien im Vergleich zu unbehandelten Tieren zu messen. Das Proflavin wird aber nicht iontophoretisch eingeführt.

Aus der Literaturstelle Chem.-Biol.Interact., Vol.42, Nr.1, Oktober 1982, S. 97-105 ist bekannt, daß Proflavin und einige seiner Derivate sich an DNA binden. Die Versuche wurden in Kulturen von Salmonella typhimurium sowie in Kulturen von L1210-Leukämiezellen durchgeführt. Über eine spezielle Art der Einbringung des Proflavins in die Zellen ist jedoch nichts angegeben.

Aus der Literaturstelle Yale J.Biol.Med., Vol. 46, Nr.5, Dezember 1973, S. 464-470 ist es bekannt, daß Proflavin und Ethidiumbromid bei Hamstermelanomen tumorspezifische Wechselwirkungen zeigen. Die Versuche wurden in vivo durchgeführt.

Aus der Literaturstelle Mol.Pharmacol., Vol. 20, Nr.2, September 1981, S.404-414 ist es bekannt, daß die Antitumor-Arzneimittel 4'-(Acridinylamino)-methansulfon-m-Anisidid und verwandte Acridine mit Nukleinsäuren in Wechselwirkung treten, wobei DNA von Kälberthymus verwendet wurde. Die Versuche wurden nur in vitro durchgeführt.

Aus der Literaturstelle Anti-Cancer Drug Des., Vol. 1, Nr.4, April 1987, S. 281-289 ist die Hemmung von Thymidylatsyntase in intakten L 1210-Zellen durch ara-C, Daunomycin, Hydroxyharnstoff und 3,4-Dihydroxybenzylamin bekannt. Auch diese Versuche wurden nur in vitro durchgeführt.

In der Literaturstelle Pharm. Acta Helv., Vol. 51, Nr.6. 1976, S.160-163 ist der Einfluß der Formulierung auf die perkutane Absorption von Proflavin-Hemisulfat beschrieben. Über die iontophoretische Einbringung in Tumorgewebe finden sich jedoch keine Angaben.

Aus der Literaturstelle Cancer Treatment Reports, Vol. 67, No. 4, April 1983, s. 391-392 ist die Verwendung von Amsacrin (Amsa), einem Acridinderivat, für die Behandlung von Blasencarcinom bekannt. Das Amsacrin wurde den Patienten intravenös verabreicht, wobei eine gewisse Antitumoraktivität bei Patienten mit metastatischem Blasencarcinom festgestellt wurde, die zuvor eine extensive Chemotherapie erhalten hatten. Da das Amsacrin jedoch systemisch angewendet wurde, waren hierfür verhältnismäßig hohe Dosen erforderlich, so daß die Nebenwirkungen überwogen. Auch hier wurde keine iontophoretische Behandlung durchgeführt.

Aus der Literaturstelle Cancer Treatment Reports, Vol.69, No.2, Februar 1985, S. 189-194 sind Vergleichsversuche zwischen Cyclophosphamid, Doxorubicin und Cisplatin (CAB) einerseits und Amsacrin andererseits, bei Patienten mit transitierendem Zellcarcinom der Harnblase bekannt. Die Mittel wurden systemisch verabreicht, und es wurde kein signifikanter Unterschied zwischen Ansacrin und Cisplatin gefunden.

Schließlich beschreibt die Literaturstelle CA 97: 207877g die iontophoretische Behandlung von colorectalen Tumoren mit Hilfe eines Doppel-Ballon-Katheters, wobei 5-Fluoruracil oder Neocarcinostatin intraluminal appliziert werden.

Beide Verbindungen fallen nicht in die Gruppe der Aminosubstituierten Heterocyclen, die in einer tautomeren Diiminform vorliegen können.

Es wurde nun gefunden, daß Heterocyclen des bei Lettré a.a.0. angegebenen Typs bei malignen Tumoren erst dann ihre antineoplatische Wirkung entfalten, wenn sie auf iontophoretischem Wege in das Tumorgewebe eingebracht werden oder wenn nach herkömmlicher lokaler Einbringung, z.B. durch Injektion, Gleichstrom (Iontophorese) angewendet wird.

Der antineoplastische Effekt ist selektiv, gesundes Gewebe wird nicht geschädigt.

Gegenstand der Erfindung ist somit die Verwendung von dissoziierbaren Heterocyclen aus der Gruppe der Aminoacridine, sowie ihrer am Grundskelett substituierten Derivate, die in einer tautomeren Diiminform vorliegen, zur Herstellung eines Arzneimittels für die iontophoretische Behandlung und Rezidivprophylaxe des Blasenkrebses sowie für die iontophoretische Behandlung bösartiger Haut- u. Schleimhautgeschwülste.

Die der Erfindung zugrundeliegende Problematik wird nachstehend am Beispiel des Blasencarnicoms erläutert.

Dem Blasencarcinom liegt eine polychronotope neoplastische Diathese der gesamten Blasenschleimhaut zugrunde. Jeder ihrer Abschnitte kann in zeitlicher Staffelung Ausgangspunkt für eine Tumorbildung sein. In den meisten Fällen sind zum Zeitpunkt der Erstdiagnose und -therapie cystoskopisch diagnostizierbare Tumorbezirke mit makroskopisch noch nicht erkennbaren generativen Tumorzellen vergesellschaftet. Diese Tatsache erklärt die extrem hohe Rezidivhäufigkeit des Blasencarcinoms. Bei diesen späteren Tumoren handelt es sich somit nicht um eigentliche "Rezidive", sondern um Neumanifestationen im Bereich der ubiquitär und potentiell kranken Schleimhaut, ausgehend von diagnostisch und therapeutisch nicht erfaßten Tumorherden. Diese spezielle Konstellation der Geschwulstbildung macht das eigentliche diagnostische, therapeutische und prognostische Dilemma der Blasenkrebserkrankung aus.

Um diesem Dilemma zu entrinnen, hat man versucht, eine lokale Chemotherapie mit bestimmten Cytostatica zur Rückbildung von Resttumoren und zur Rezidivprophylaxe nach erfolgten Tumorentfernungen, z.B. durch transurethrale Elektroresektionen, durchzuführen. Das geschieht dadurch, daß man diese Stoffe in wäßrigen Lösungen über einen Katheter in die Blase einbringt und dort etwa zwei Stunden beläßt.

Diese lokale Chemotherapie war jedoch zur Beseitigung von Resttumoren oder zur Rezidivprophylaxe nicht sehr erfolgreich, da die Blase normalerweise kein resorptives Organ ist. Die oberflächliche Zellschicht der Schleimhaut stellt eine Membran dar, die ionische Substanzen nicht passieren können. Nicht-ionische Verbindungen können die Membran unter bestimmten Voraussetzungen überwinden, weshalb die für die lokale Chemotherapie verwendeten Präparate nicht-ionische Cytostatica sind. Sie müssen ein Molekulargewicht über 200 haben, da Substanzen mit niedrigem Molekulargewicht gleich nach Überwindung der Membran von den Blutgefäßen erfaßt und abtransportiert werden. Je niedriger das Molekulargewicht ist, desto oberflächlicher ist die cytostatische Wirkung in der Tiefe der Blasenwand, und umso näher steht diese lokale Chemotherapie der systemischen Chemotherapie in Bezug auf Nebenwirkungen. Die lokale Chemotherapie der Blase hat also die Nachteile jeder Chemotherapie. Diese Medikamente sind zudem nicht selektiv, können gesunde von kranken Zellen nicht unterscheiden, schädigen die Schleimhaut, machen subjektive Beschwerden und können selbst zu Krebsgeschwülsten führen.

Die erfindungsgemäße Verwendung der vorstehend definierten Heterocyclen, die in dissoziierbarer, d.h. in Lösung in ionischer Form vorliegen, zur Herstellung von Arzneimitteln zur iontophoretischen Tumorbehandlung eröffnet nun die Möglichkeit einer schonenden Behandlung, d.h. einer hochkonzentrierten regionalen antineoplastischen Behandlung in allen Blasenschichten ohne systemische Nebenwirkungen und damit eine Tumorrückbildung bei gleichzeitiger Rezidiv-Prophylaxe.

Unter "Iontophorese" versteht man erfindungsgemäß die Einwanderung der in dissoziierter Form, d.h. in Ionenform vorliegenden Heterocyclen über die Haut und Schleimhaut in den Tumor unter der Einwirkung von Gleichstrom. Die Iontophorese ist jedoch ein verhältnismäßig komplexer Vorgang, an dem noch zusätzliche Phänomene, wie Elektrophorese, Elektroosmose, Elektrolyse und Diffusion beteiligt sind. Der Gleichstrom bewirkt eine gute und schnelle Verteilung des Heterocyclus im Tumorgewebe. Ferner tritt bei der Iontophorese wahrscheinlich noch ein Stromeffekt dahingehend auf, daß die elektrischen Doppelschichten der Zellmembranen bzw. Zellkernmembranen neutralisiert werden, wodurch die Sperrwirkung dieser Membranen gegenüber den in ionisierter Form vorliegenden Heterocyclen aufgehoben wird. Ein weiterer spezieller Stromeffekt besteht darin, daß der Heterocyclus in der Tumorzelle bzw. im Tumorzellkern unter Gleichstrom eine Zellteilungshemmung mit konsekutivem Zellenuntergang (Tumorrückbildung) bewirkt. Diese Wirkung ist selektiv auf die Tumorzellen gerichtet und schädigt gesundes Körpergewebe nicht.

Erfindungsgemäß werden dreikernige Heterocyclen verwendet, die neben dem Ringstickstoffatom noch eine oder mehrere Aminogruppen enthalten.

Diese Verbindungen liegen in Salzform vor, wobei der wirksame Teil des Heterocyclus das Kation bildet.

Der Heterocyclus liegt vorzugsweise als Mono- oder Dichlorid, -sulfat,-lactat oder -acetat vor, wobei der bevorzugt verwendete Heterocyclus das Proflavin-Monohydrochlorid ist, das chemisch als 3,6-Diaminoacridin-Hydrochlorid bezeichnet wird und das die nachstehend angegebene Formel hat: 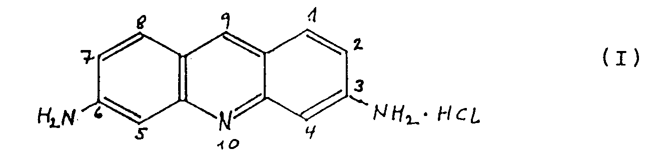
Die tautomere Diiminform kann durch die nachstehend angegebene Formel dargestellt werden 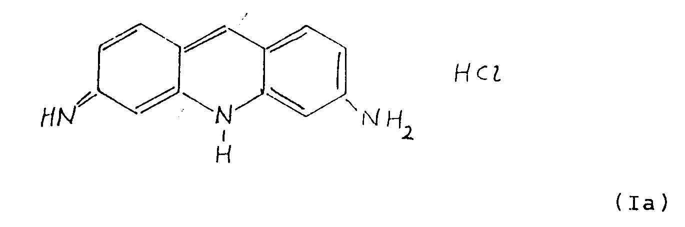
Ein weiteres Beispiel für einen erfindungsgemäß verwendeten Heterocyclus ist das Trypaflavin oder Acriflaviniumchlorid (internationaler Freiname für die Mischung der Hydrochloride von 3,6-Diamino-10-methylacridiniumchlorid (vgl. nachstehend angegebene Formel) 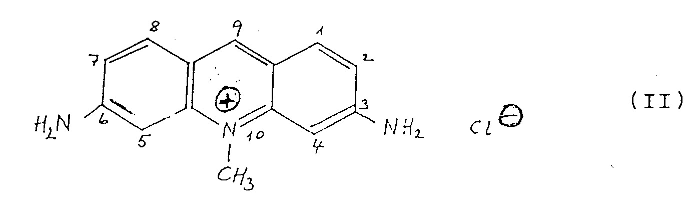
und 3,6-Diaminoacridin.

Die erfindungsgemäß verwendeten Heterocyclen können am Grundskelett, d.h. sowohl am Ringstickstoffatom als auch an den Ringkohlenstoffatomen durch Substituenten, wie Halogene, Alkyl- oder Alkoxygruppen (insbesondere mit 1 bis 4 Kohlenstoffatomen), Arylgruppen, Alkarylgruppen und Aralkylgruppen (insbesondere in denen die Arylgruppe eine Phenylgruppe darstellt) substituiert sein.

Die erfindungsgemäß verwendeten Heterocyclen wirken bei der Iontophorese selektiv auf die Tumorzellen. Dies beruht mit großer Wahrscheinlichkeit auf einem unterschiedlichen Membranverhalten von Tumor- und gesunder Zelle, besonders der Kerne und Mitochondrien, da es unter dem Einfluß des elektrischen Stromes nur in der Tumorzelle zu einer Anlagerung der Heterocyclen an die DNA als Voraussetzung für einen irreparablen Zelluntergang kommt.

Nach einer bevorzugten Ausführungsform liegen die erfindungsgemäß verwendeten Heterocyclen im Gemisch mit Dimethylsulfoxid (DMSO) in wäßriger Lösung vor. In Verbindung mit der Iontophorese kommt es durch das DMSO zu einer Erhöhung der transportierten Heterocyclus-Wirkstoffmenge um das Dreifache gegenüber einem Blaseninstillat ohne DMSO. DMSO allein ist jedoch nicht in der Lage, diese als Membran wirkende oberflächliche Zellschicht der Blasenschleimhaut für ionische Substanzen wie es die vorliegenden Heterocyclen sind, permeabler zu machen und eine stärkere Anreicherung der Heterocyclen in der Blasenwand zu ermöglichen. Zum anderen bewirkt das DMSO eine bessere Verteilung der Heterocyclen im Gewebe, die für die antineoplastische Erfassung aller Tumorzellen von großer Wichtigkeit ist.

Das DMSO hat bei der Behandlung von Blasencarcinomen außerdem eine lokale analgetische und spasmolytische Wirkung, wodurch bei den angewandten Stromdichten Iontophoresen über einen längeren Zeitraum möglich sind.

Üblicherweise liegt der erfindungsgemäß verwendete Heterocyclus in wäßriger Lösung in einer Konzentration von 0,05 g/l bis zur Löslichkeitsgrenze, vorzugsweise bis zu 5 g/l, vor. Das Dimethylsulfoxid liegt vorzugsweise in wäßriger Lösung in einer Konzentration von 20 bis 300 g/l vor.

Eine bevorzugte Herrichtung oder Rezeptur enthält Proflavin-Monohydrochlorid in einer Konzentration von 0,05 bis 5 g/l und DMSO in einer Konzentration von 20 bis 300 g/l. Insbesondere enthält die Rezeptur 1 g/l Proflavin-Monohydrochlorid und 150 g/l Dimethylsulfoxid. Mit Hilfe einer derartigen Rezeptur kann die Iontophorese in einer Sitzung auf 1/2 Stunde und länger ausgedehnt werden.

Die iontophoretische Behandlung erfolgt vorzugsweise mit einem Gleichstrom, wobei die auf den Körperflächen bzw. den Tumor angewandten Stromdichten nicht über 0,5 mA/cm² liegen. Die Stromdichte beträgt vorzugsweise 0,3 bis 0,46 mA. Unter dem Begriff "Gleichstrom" versteht man auch pulsierende Gleichströme sowie bestimmte Arten von Wechselströmen, bei denen eine Halbwelle eine größere Amplitude als die andere hat bzw. bei denen die Integralfläche unter einer Halbwelle größer ist als unter der anderen Halbwelle.

Bei der Iontophorese kommt es zu einer Wanderung der als Kationen vorliegenden Heterocyclen in das Körpergewebe und in die Tumorzellen. Des weiteren findet - wie schon erwähnt - nach Überwindung der Membranstrukturen vorwiegend der Kerne und Mitochrondrien eine Interaktion der Heterocyclen mit der DNA der Tumorzellen statt, die beim gesunden Gewebe ausbleibt. Dieses Phänomen macht die selektive antineoplastische Wirkung der Heterocyclen unter der Iontophorese aus.

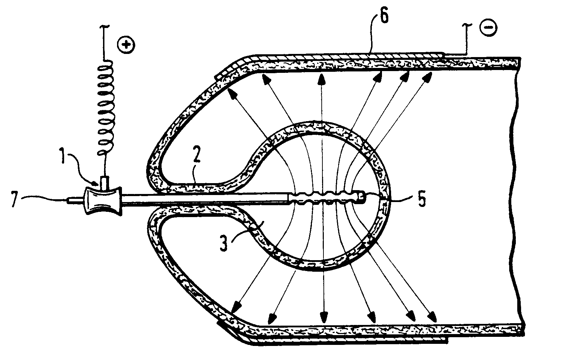

Bei der Behandlung von Blasencarcinomen wird das Medikament vorzugsweise mit Hilfe einer rohrförmigen positiven Elektrode 1, wie sie in der Zeichnung dargestellt ist, durch die Harnröhre 2 in die Blase 3 eingeführt. Die positive Elektrode ist im Bereich der Harnröhre isoliert, im Bereich der Blase mit Öffnungen 4 versehen und am distalen Ende 5 mit einer Kappe aus isolierendem Material versehen. Die negative gürtelförmige Außenelektrode ist in der Zeichnung mit 6 bezeichnet. Die Pfeile markieren die Stromrichtung bzw. den Wirkstoffstransport aus dem Blasenlumen in die Blasenwand und zur negativen Außenelektrode. Das Blaseninstillat, das bei 7 eingeführt wird, ist ein Gemisch aus destilliertem Wasser, Heterocyclus (insbesondere Proflavin-Monohydrochlorid) und DMSO in dem vorstehend angegebenen Konzentrationsbereich.

Die Blase ist für die Iontophorese ein ideales Ausnahmeorgan, da sie die Aufnahme einer Elektrolytlösung, d.h. einer Lösung des in Ionenform vorliegenden Heterocyclus, und dessen gezielte Einschleusung und Penetration in die Blasenwand gestattet. Theoretisch ist die transportierte und die aus dem Instillat in die Blasenwand eingebrachte Menge des Heterocyclus das Produkt aus Zeit mal Stromstärke; in der Praxis muß jedoch mitberücksichtigt werden, daß während der iontophoretischen Behandlung mit dem Urin Fremdionen in das Instillat gelangen, die mit den zu transportierenden Heterocyclus-Ionen konkurrieren. Deshalb muß die verwendete Behandlungslösung frei von Fremdionen sein und eine genügend hohe Heterocyclus-Konzentration haben, um zu verhindern, daß der Stofftransport vorwiegend durch die Fremdionen erfolgt.

Schon nach einer Behandlungsdauer von 30 min. kann der eingeschleuste Wirkstoff in allen drei Blasenwandschichten in hohen Konzentrationen nachgewiesen werden, was ohne Anwendung der intravesikalen Iontophorese bisher noch nicht erreicht werden konnte.

Das erfindungsgemäß bevorzugt verwendete Proflavin (in Kationenform) zeichnet sich durch eine gute Gewebeverträglichkeit aus. In therapeutischen Dosen (1 g/l) reizt Proflavin das Gewebe nicht und ruft auch keine allergischen Erscheinungen hervor. Es ist unabhängig vom pH-Wert wirksam und zeichnet sich durch eine gute chemische Stabilität aus. Man findet einen extremen bakteriostatischen und bakteriziden Effekt in Lösungen niedrigster Proflavin-Konzentration.

Bei der iontophoretischen Behandlung des Blasenkrebses wurde festgestellt, daß beim Einführen der Elektrode 1 durch die Harnröhre in die Blase in einigen Fällen Komplikationen auftraten, wenn kein sogenanntes Gleitmittel verwendet wurde. Gleitmittel werden bei der Diagnose bzw. bei der Therapie von Blasenerkrankungen (Blasenspiegelungen bzw. Elektroresektionen) bereits dazu verwendet, um die hierfür erforderlichen Instrumente komplikationslos in die Harnröhre einführen zu können. Diese Gleitmittel unterschiedlichster Zusammensetzung (Gele, Emulsionen) sind zudem meist mit einem Schleimhautanästhetikum versetzt, um die Instrumenteneinführung weitgehend schmerzlos zu gestalten.

Beim elektrischen Operieren in der Blase durch die Harnröhre, z.B. mittels Hochfrequenzströmen und mit Instrumenten, die einen Metallschaft aufweisen, werden Gleitmittel mit hoher Leitfähigkeit (etwa 7 mS/cm) verwendet, während bei Geräten mit einem isolierten Schaft (der Schaft liegt hierbei in der Harnröhre) Gleitmittel mit geringer Leitfähigkeit (etwa 1 bis 2 mS/cm) benutzt werden. Dadurch können starke Schmerzen in der Harnröhre, schwere elektrische Harnröhrenreizungen und -verletzungen (Verbrennungen, Strikturen) auf dem Boden von Fehl- oder Leckströmen vermieden werden.

Wenn man die verwendete Elektrode 1 mit Hilfe eines mit einem Lokalanästhetikum versetzten ionisch leitfähigen handelsüblichen Gleitmittels durch die Harnröhre in die Blase einführt, so geschieht folgendes:
A) Bei der Einbringung des Elektrode durch die Harnröhre in die Harnblase gelangt ein Teil des als Elektrolytgemisch vorliegenden Gleitmittels auch in die Harnblase. Dort konkurriert es in Form von sogenannten Fremdionen mit dem ionischen Heterocyclus, dessen Transport in die Blasenwand und in den Tumor und dessen antineoplastische Wirkung dadurch deutlich geringer bzw. schwächer werden.
B) Schon bei geringem Spasmus der Blase kann aus dem therapeutischen Blaseninstillat des disoziierbaren Heterocyclus während der laufenden Iontophorese eine geringe Flüssigkeitsmenge in die Harnröhre eindringen. Sie ist ionisch leitend und setzt in diesem Fall zusammen mit dem ebenfalls leitenden Gleitmittel die empfindliche Harnröhrenschleimhaut direkt dem Strom aus. Je unregelmäßiger die Stromverteilung (unterschiedlich starke Gleitmittelschichten) desto größer sind die punktuellen Stromdichten weit über die Verträglichkeitsgrenzen hinaus, wodurch Schleimhautschädigungen bis hin zur Kolliquationsnekrose entstehen. Die unmittelbare Folge noch unter der Iontophorese können, je nach dem Grad der erzielten Anästhetisie, Mißempfindungen oder Schmerzen sein. Das kann zu schmerzhaften Blasenkontraktionen führen, die die Blasenkapazität herabsetzen und das therapeutische Blaseninstillat neben der Sonde abfließen lassen und schließlich die vorzeitige Beendigung der Behandlung herbeiführen. Ist die Anästhesie im anderen Fall ausreichend, unterbleibt die eben geschilderte Symptomatik, und die Iontophorese wird zeitgerecht zu Ende geführt. Nach mehreren Stunden bis nach einem Tag setzt jedoch die Beschwerdesymptomatik meist akut und heftig ein (schmerzhafte Pollakisurie, starkes Brennen in der Harnröhre usw), die die erforderliche zweite Sitzung innerhalb eines Behandlungszyklus im Abstand von sieben Tagen unmöglich machen kann. Die Symptomatik ist weitgehend therapieresistent und kann in abnehmender Intensität bis zu 4 Wochen andauern. Von Zyklus zu Zyklus verstärkt sie sich, wodurch die weitere iontophoretische Behandlung überhaupt und damit der gesamte Therapieerfolg infragegestellt werden.

Diese Problematik tritt insbesondere bei Patienten auf, deren Blase durch lange und herkömmliche Behandlungsmaßnahmen (Elektroresektion, lokale Anwendung von Cytostatika) schwer geschädigt ist und sich in einem ganz empfindlichen Zustand (Spasmusneigung) befindet.

Um die vorstehend geschilderte Problematik zu vermeiden und eine effektive und schmerzlose iontophoretische Behandlung des Blasenkrebses ermöglichen, wird daher vorzugsweise ein Gleit-, Anästhesie- und Schutzmittel verwendet, das
a) ein nichtionisches, mit Wasser mischbares Gleitmittel mit einer elektrischen Leitfähigkeit von weniger als 2 mS/cm und einer Viskosität (bei 25°C) im Bereich von 50 bis 2000 mPa.s und
b) ein nicht in den Blutkreislauf eintretendes lokales, nichtionisches Schleimhautanästhetikum enthält,
wobei das Gemisch aus (a) und (b) einen pH-Wert zwischen 5 und 7,5 hat.

Dieses Mittel ist aufgrund seines pH-Wertes von 5 bis 7,5, vorzugsweise von 6,5 bis 6,8, gut verträglich. Es darf die angegebene elektrische Leitfähigkeit nicht überschreiten, wobei seine Leitfähigkeit vorzugsweise weniger als 1, insbesondere weniger als 0,5 mS/cm betragen soll; es muß zudem über eine ausreichende Viskosität verfügen, damit ein durchgehender Film zwischen der Wand der Harnröhre und der eingeführten Elektrode erhalten bleibt.

Vorzugsweise liegt seine Viskosität (bei 25°C) im Bereich von 100 bis 1500 mPa.s.

In seiner Funktion als Vorbehandlungsmittel ermöglicht das Mittel einmal die schmerzlose und atraumatische Einführung der Elektrode durch die Harnröhre in die Blase und zum anderen schützt es die Harnröhre vor elektrischen Leckströmen während der iontophoretischen Behandlung. Außerdem werden bei unbeabsichtigtem Abfluß einer überschüssigen Menge des Mittels keine Fremdionen in das therapeutische Blaseninstillat (d.h. des in ionischer Form vorliegenden Heterocyclus) abgegeben und dadurch eine Effektivitätsabschwächung des Transportes bzw. der antineoplastischen Wirkung des Heterocyclus verhindert.

In seiner Funktion als Nachbehandlungsmittel, d.h. wenn es nach der Beendigung der Iontophorese über die noch liegende Elektrode in die Harnblase eingebracht wird, bewirkt das Mittel eine Oberflächenanästhesie der gesamten Harnblasenschleimhaut und dadurch die Vermeidung von nach der Behandlung auftretenden Blasenspasmen mit konstriktiver Pollakisurie und Schmerzen, die die Durchführung einer notwendigen weiteren Behandlung in einem zeitlichen Abstand von nur wenigen Tagen unmöglich machen könnten.

Die Verwendung eines üblichen Lokalanästhetikums, wie Lidocain, wäre in diesem Fall kontraindiziert, weil ein solches Medikament sehr schnell durch die Blasenwand resorbiert und systemisch werden würde. Bei den in Bezug auf die große Blasenoberfläche erforderlichen wirksamen Mengen bestünde eine letale Vergiftungsgefahr.

Bei dem Gleitmittel (a) handelt es sich vorzugsweise um einen Polyol oder ein Polyolderivat, die mit Wasser mischbar sind, z.B. Diethylenglykol, Triethylenglykol und höhere Polyethylenglykole. Auch Triole und höhere Polyole sowie deren veretherte Derivate sind brauchbar. Besonders bevorzugt wird Glycerin verwendet.

Das Lokalanästhetikum, bei dem es sich ebenfalls um eine lokal gewebsfreundliche und nichtionische Substanz handelt, ist vorzugsweise ein Polyethylenglykolether mit einem Molekulargewicht von ≧ 450. Insbesondere stellt das Lokalanästhetikum einen Polyethylenglykolmonododecylether mit der Formel

H₃C-(CH₂)₁₁-(O-CH₂-CH₂)ₓ-OH

dar, worin x eine Zahl zwischen 6 und 12 bedeutet. Diese Verbindung wird kurz mit dem internationalen Freinamen Polidocanol bezeichnet.

Das Gleit-, Anästhesie und Schutzmittel zeichnet sich insbesondere dadurch aus, daß das Gewichtsverhältnis zwischen Gleitmittel (a) und Lokalanästhetikum (b) etwa 10 bis 200 : 1, vorzugsweise etwa 15 bis 100 : 1, insbesondere 20 : 1 beträgt.

Vorzugsweise liegt das Mittel in Form einer etwa 0,5 bis 7-Gew.-%igen, insbesondere einer etwa 5-Gew.-%igen Lösung von Polidocanol in Glycerin vor.

Die Erfindung ist durch die nachstehenden Beispiele erläutert:

### Beispiel 1

### Intravesikale Iontophorese mit Proflavin-Monohydrochlorid

Es wurde eine Blaseninstillation mit 200 ml einer 0,1%igen Proflavin-Monohydrochloridlösung vorgenommen, wobei keine wesentlichen subjektiven und objektiven lokalen Reizerscheinungen auftraten. Die Behandlungsdauer betrug 30 min, die verwendete Stromstärke 50 mA, woraus sich rechnerisch im Bereich der gesamten Blasenwand (Oberfläche) eine Stromdichte von 0,3 mA/cm² ergab.

Nach der Behandlung wurden in den drei Blasenschichten folgende Konzentrationen an Proflavin-Monohydrochlorid-Lösung gefunden:

| | | |
|---|---|---|
| (I) | Schleimhaut: | 5.20 µg/g, |
| (II) | Muskularis | 13.80 µg/g |
| (III) | perivesicales Gewebe | 2.10 µg/g |

Zum Vergleich wurde eine entsprechende Blaseninstillation ohne Iontophorese vorgenommen. Hierbei kam es nicht zum Übertritt des Arzneimittels in die Blasenwand, was durch eine Probeexzision in Verbindungmit einer Flüssigkeitschromatographie festgestellt wurde.

Die iontophoretisch erreichten Proflavin-Gewebsspiegel machen einerseits deutlich, daß die Substanz nach dem Passieren der sonst von ionisierten Verbindungen nicht überwindbaren superficialen und als Membran wirkenden Zellschicht der Blasenschleimhaut nicht sofort Anschluß an das Gefäßsystem findet und abtransportiert wird, sondern sich in der ganzen Blasenwand verteilt und andererseits, das gerade in der Muscularis (Schicht II) das Maximum der Proflavin-Konzentration besteht. Das ist besonders wichtig für die Behandlung der wandinfiltrierenden Formen des Blasencarcinoms.

### Beispiel 2

### Iontophoretische Behandlung von Modell-Hauttumoren mit Proflavin-Monochlorid bei Ratten

Es wurden Walker-Tumoren am Rücken von 300 g schweren Lewis-Ratten als Modell-Hauttumore präpariert. 7 Tage nach der Impfung hatten sich walnußgroße Geschwülste entwickelt. Es wurden zwei Versuchsgruppen gebildet. Den Tieren der ersten Gruppe wurden in die sieben Tage alten walnußgroßen Tumoren je eine Injektion von jeweils 0,45 ml einer 2%igen Proflavin-Monohydrochlorid-Lösung (= 9 mg) in Abständen von 6 Tagen verabreicht. Die Geschwülste entwickelten sich explosionsartig weiter und waren beim Exitus 7 Tage nach der ersten Injektion nach einem schweren Krankheitsverlauf hühnereigroß. Nach der einschlägigen Literatur beträgt die statistische Überlebenszeit der Walker-Ratten auch in Abhängigkeit von der verimpften Zellzahl, durchschnittlich 19 Tage (verimpfte Zellzahl für die in diesem Versuch erzeugten Tumore: 1 Mio).

In der zweiten Gruppe mit ebenfalls 7 Tage alten walnußgroßen Tumoren erfolgten drei intratumorale Injektionen in gleicher Dosierung wie oben in 6-tägigen Abständen, denen sich jeweils iontophoretische Anwendungen mit kleinflächigen Elektroden anschlossen (30 min. pro Sitzung bei 0,2 bis 0,3 mA/cm² Tumorfläche). Sofort nach der ersten Sitzung war das Tumorwachstum gestoppt. Die Tiere zeigten ein gutes Allgemeinbefinden und eine Gewichtszunahme während des gesamten weiteren Verlaufs. Am Tage der dritten und letzten Sitzung war der Tumor bereits um die Hälfte verkleinert. Nach weiteren 14 Tagen war an der Stelle des ehemaligen Tumorsitzes keine Geschwulst mehr nachweisbar. Die Tiere überlebten ohne Tumorrezidiv ein Jahr und länger.

Fast identische Resultate wurden durch Verwendung von Trypaflavin-Hemisulfat erzielt.

Der Versuch steht für die klinische Übertragung der Versuchsanordnung auf menschliche Hauttumore.

Die Ergebnisse dieses Versuches wurden durch die Resultate eines weiteren Versuches bestätigt:

### Beispiel 3

### Iontophoretische Behandlung von Modell-Hauttumoren mit mit Proflavin-Monochlorid bei Mäusen

Auf 30 Nacktmäuse (20 g Körpergewicht) wurden als Modell-Hauttumore menschliche Pankreastumoren (Impftumore) im Rückenbereich transplantiert. Dieses Tumormodell ist therapieresistent und nicht durch Cytostatika zu beeinflussen.

In der ersten Gruppe (15 Mäuse) wurden in vier Sitzungen in ca. 3 bis 5-tägigen Abständen je nach Tumorgröße zwischen 0,25 und 0,75 mg Proflavin-Monohydrochlorid in Form einer verdünnten wäßrigen Lösung intratumoral eingespritzt. Nach den Injektionen erfolgten über jeweils 30 min. iontophoretische Behandlungen mit den Elektroden nach Beispiel 2 (Stromdichte 0,2 bis 0,3 mA/cm²). Vier Tiere verstarben an interkurrenten Krankheiten, die nicht im Zusammenhang mit der Tumorerkrankung bzw. -behandlung standen. Die Tumore der übrigen Tiere bildeten sich vollständig und defektfrei zurück.

In der ebenfalls 15 Tiere umfassenden Kontrollgruppe wurde die gleiche Proflavin-Monohydrochlorid-Menge in denselben zeitlichen Abständen verabreicht, wie in der Behandlungsgruppe, jedoch unterblieben die lokalen Iontophoresen. Alle Tiere starben durch fortschreitendes Tumorwachstum an Kachexie.

Der Versuch steht für die klinische Übertragung der Versuchsanordnung auf menschliche Schleimhauttumore.

### Beispiel 4

### Klinische Behandlung mit Proflavin-Monohydrochlorid

### W. Sch., männlich, 67 Jahre

Es lag ein multilokullär rezidivierendes Blasencarcinoms vor, das innerhalb von etwa 3 Jahren andernorts erfolglos durch transurethrale Elektroresektionen und eine fast zweijährige lokale kontinuierliche Cytostatika-Anwendung vorbehandelt worden war. Bei der stationären Aufnahme bestanden schwerste Blasentenesmen mit einer fünfminütigen Miktionsfrequenz, einem himbeerfarbenen Urin und häufigen Koagelabgängen.

Es wurden 5 intravesicale Proflavin-Iontophoresen mit 0,1%igem Proflavin-Monohydrochlorid als Blaseninstillat über jeweils eine halbe Stunde bei 50 mA durchgeführt. Danach erfolgte eine rasche Sistierung der Hämaturie und eine allmähliche Rückbildung der Tenesmen. 110 Tage nach Behandlungsbeginn ergab sich makroskopisch und histologisch kein Anhalt mehr für einen Tumor. Zwei Monate später fand sich bei Beschwerdefreiheit cystoskopisch eine völlig reizlose Blasenschleimhaut ohne Hinweis auf ein Carcinom-Rezidiv.

### Beispiel 5

### Behandlung mit Proflavin-Monochlorid/DMSO

### D.M., weiblich, 77Jahre

### Diagnose

Ausgeprägtes infiltrierendes Blasencarcinom mit Harnstauung beiderseits (Ersterkrankung).

### Zur Behandlung verwendetes Präparat

1 g Proflavin-Monohydrochlorid, 150 g DMSO, 850 ml dest. Wasser.

### Behandlung und Verlauf

Es wurden insgesamt 6 intravesicale Iontophoresen von jeweils einstündiger Dauer in Abständen von 7 Tagen unter Wechsel der Lösung bei 50 mA ohne Narkose und ohne Komplikationen durchgeführt. Bei der cystoskopischen Kontrolle am siebten Tag nach Behandlungsbeginn zeigte sich bereits eine deutliche Abflachung der tumorösen Veränderungen und eine Schrumpfung der knolligen Tumorsubstrate. Am 33. Tag nach Behandlungsbeginn war nur noch ein apfelsinenkerngroßer Tumorrest im Trigonum festzustellen. Die Patientin wurde nach 43 Tagen in gutem Allgemeinzustand bei urologischer Beschwerdefreiheit und ohne spezielle Blasensymptomatik entlassen.

Das bevorzugt verwendete Gleit-, Anästhesie- und Schutzmittel sowie dessen Anwendung sind in den nachstehenden Beispielen erläutert.

### Beispiel 6

5 g Polidocanol (MG 600) werden mit 95,5 g wasserfreiem Glycerin vermischt, wodurch ein viskoses Gemisch mit einer elektrischen Leitfähigkeit von ≦ 0,1 mS/cm erhalten wird.

### Beispiel 7

10 ml der nach Beispiel 6 erhaltenen Lösung wurden durch eine mit einer Olive bestückten Injektionsspritze in die Harnröhre eines männlichen Blasenkrebspatienten instilliert. Nach etwa 10 Minuten trat die analgetische Wirkung ein, worauf eine positive Elektrode 1, wie sie vorstehend beschrieben ist, eingeführt wurde, wobei der Patient keine Schmerzen verspürte und keine Verletzungen in der Harnröhre gesetzt wurden. Der außen nicht leitende Schaft der Elektrode war von einem zusammenhängenden Film des erfindungsgemäßen Mittels isolierend umgeben. Durch die so plazierte Elektrode wurden etwa 150 ml einer wäßrigen Lösung von Proflavin-Monohydrochlorid (0,1 Gew.-%) und Dimethylsulfoxid (15 Gew.-%) eingeführt. Die Ionthophoresedauer betrug 60 Minuten bei 40 mA, was einer Stromdichte im Bereich der Blasenschleimhaut von etwa 0,3 mA/cm² entsprach.

Nach der Iontophorese wurde das therapeutische Blaseninstillat (Proflavin-Monohydrochloirid, DMSO und Wasser) abgelassen, und die Blase wurde mit destilliertem Wasser gespült.

Dann wurden durch die Elektrode 20 ml der Lösung von Beispiel 6 als Nachbehandlungsmittel direkt in die Blase instilliert, worauf die Elektrode entfernt wurde. Das Mittel verblieb bis zur natürlichen Entleerung in der Blase.

Die Wirkung dieser Nachbehandlung äußerte sich in einer deutlichen Herabsetzung der Miktionsfrequenz sowie in einer weitgehenden Vermeidung von Blasenspasmen, die ansonsten zu einer funktionellen Minderung der Blasenkapazität führen würden.

Aufgrund dieser Behandlung blieb die erforderliche Blasenkapazität für erforderliche weitere iontophoretische Behandlung erhalten.

Wenn das Mittel von Beispiel 6 bei einem anderen Patienten, der durch eine herkömmliche Behandlung eine hochgeschädigte Blase hatte, nicht verwendet wurde, so klagte dieser während der Iontophoresebehandlung über Schmerzen in der Harnröhre. Ein Teil des Blaseninstillats wurde neben der in der Harnröhre liegenden Elektrode durch einen Blasenspasmus nach außen gepreßt. Die weitere iontophoretische Behandlung dieses Patienten mußte bis zum Abklingen der negativen Symptomatik (Komplikation) für etwa 4 Wochen unterbrochen werden.

## Patentansprüche

1. Verwendung von dissoziierbaren Heterocyclen aus der Gruppe der Aminoacridine sowie ihrer am Grundskelett substituierten Derivate, die in einer tautomeren Diiminform vorliegen, zur Herstellung eines Arzneimittels für die iontophoretische Behandlung und Rezidivprophylaxe des Blasenkrebses sowie für die iontophoretische Behandlung bösartiger Haut- u. Schleimhautgeschwülste.

2. Verwendung nach Anspruch 1, dadurch gekennzeichnet, daß der Heterocyclus in Salzform vorliegt.

3. Verwendung nach Anspruch 1 und 2, dadurch gekennzeichnet, daß der Heterocyclus als Mono- oder Dichlorid, -sulfat, -lactat oder -acetat oder in Form eines anderen physiologisch verträglichen Salzes vorliegt.

4. Verwendung nach einem der Ansprüche 1 bis 3, dadurch gekennzeichnet, daß der wirksame Teil des Heterocyclus ein Kation ist.

5. Verwendung nach einem der Ansprüche 1 bis 4, dadurch gekennzeichnet, daß der Heterocyclus Proflavin-Monohydrochlorid ist.

6. Verwendung nach einem der Ansprüche 1 bis 5, dadurch gekennzeichnet, daß der Heterocyclus im Gemisch mit Dimethylsulfoxid (DMSO) in wäßriger Lösung vorliegt.

7. Verwendung nach einem der Ansprüche 1 bis 6, dadurch gekennzeichnet, daß der Heterocyclus in wäßriger Lösung in einer Konzentration von 0,05 g/l bis zur Löslichkeitsgrenze, vorzugsweise bis zu 5 g/l vorliegt.

8. Verwendung nach einem der Ansprüche 1 bis 7, dadurch gekennzeichnet, daß das Dimethylsulfoxid in wäßriger Lösung in einer Konzentration von 20 bis 300 g/l vorliegt.

## Claims

1. Use of dissociable heterocylic compounds from the group of aminoacridines and derivatives thereof which are substituted on the basic structure and which are present in a tautomeric diimine form, for the preparation of a medicament for the iontophoretic treatment and recidivism prophylaxis of bladder cancer, as well as for the iontophoretic treatment of malignant skin and mucous membrane tumours.

2. Use according to Claim 1, characterised in that the heterocylic compound is present in salt form.

3. Use according to Claims 1 and 2, characterised in that the heterocylic compound is present as mono or dichloride, sulphate, lactate or acetate, or in the form of another physiologically compatible salt.

4. Use according to any one of Claims 1 to 3, characterised in that the active part of the heterocylic compound is a cation.

5. Use according to any one of Claims 1 to 4, characterised in that the heterocylic compound is proflavinemonohydrochloride.

6. Use according to any one of Claims 1 to 5, characterised in that the heterocylic compound is present in a mixture with dimethyl sulphoxide (DMSO) in aqueous solution.

7. Use according to any one of Claims 1 to 6, characterised in that the heterocylic compound is present in aqueous solution in a concentration of from 0.05 g/l up to the limit of solubility, preferably up to 5 g/l.

8. Use according to any one of Claims 1 to 7, characterised in that the dimethyl sulphoxide is present in aqueous solution in a concentration of from 20 to 300 g/l.

## Revendications

1. Utilisation d'hétérocycles dissociables appartenant au groupe des aminoacridines ainsi que de leurs dérivés substitués sur le squelette de base, qui sont présents sous la forme de diimine tautomère, pour la fabrication d'un médicament pour le traitement et la prophylaxie de rechute iontophorétiques du cancer de la vessie, ainsi que pour le traitement iontophorétique de tumeurs de type malin de la peau et des muqueuses.

2. Utilisation selon la revendication 1, caractérisée par le fait que l'hétérocycle est présent sous la forme de sel.

3. Utilisation selon l'une des revendications 1 et 2, caractérisée par le fait que l'hétérocycle est présent sous la forme d'un mono- ou d'un di-chlorure, -sulfate, -lactate ou -acétate, ou sous la forme d'un autre sel physiologiquement compatible.

4. Utilisation selon l'une quelconque des revendications 1 à 3, caractérisée par le fait que la partie active de l'hétérocycle est un cation.

5. Utilisation selon l'une quelconque des revendications 1 à 4, caractérisée par le fait que l'hétérocycle est le monochlorhydrate de proflavine.

6. Utilisation selon l'une quelconque des revendications 1 à 5, caractérisée par le fait que l'hétérocycle est présent en solution aqueuse en mélange avec du diméthylsulfoxyde (DMSO).

7. Utilisation selon l'une quelconque des revendications 1 à 6, caractérisée par le fait que l'hétérocycle est présent en solution aqueuse, dans une concentration de 0,05 g/l jusqu'à la limite de solubilité, de préférence, jusqu'à 5 g/l.

8. Utilisation selon l'une quelconque des revendications 1 à 7, caractérisée par le fait que le diméthylsulfoxyde est présent en solution aqueuse, dans une concentration de 20 à 300 g/l.
